# EUROPÄISCHE PATENTANMELDUNG

(11) **EP 1 489 162 A2**
(43) Veröffentlichungstag der Anmeldung: **22.12.2004**
(21) Anmeldenummer: 04013768.9
(22) Anmeldetag: 11.06.2004
(51) Int. Cl.: C12M 1/34, C12N 5/06

(54) **Vefahren und Vorrichtung zur Bestimmung von ausdifferenzierten Säugerzellen**

(30) Priorität: 12.06.2003 DE 10326966
(71) Anmelder: Joseph, Heinz Walter, Dr., 79108 Freiburg (DE)
(72) Erfinder: Joseph, Heinz Walter, Dr., 79108 Freiburg (DE)
(74) Vertreter: Teipel, Susanne, Dr.

(57) **Zusammenfassung**

Die Erfindung betrifft ein Verfahren zur *in-vitro* Bestimmung, insbesondere Typisierung ausdifferenzierter Säugerzellen, umfassend die Schritte: a. Bestrahlen der Zellen mit Infrarot-Licht und Aufzeichnen des erhaltenen Absorptionsspektrums; b.

Durchführen einer Fourier-Transformation des Absorptionsspektrums aus (a) zum Erhalt eines FT-IR-Spektrums; c. Berechnen der ersten und gegebenenfalls höherer Ableitungen des FT-IR-Spektrums; und d. Vergleichen der Ableitung(en) aus Schritt (c) in einem vorgewählten Wellenzahlbereich mit derselben bzw. denselben Ableitung(en) zuvor ermittelter Referenz-FT-IR-Spektren, sowie eine Vorrichtung zur *in-vitro* Bestimmung, insbesondere Typisierung ausdifferenzierter Säugerzellen, umfassend: a. eine Probenaufnahmevorrichtung für eine Suspension der ausdifferenzierten Säugerzellen; b. Mittel zum Bestrahlen der Zellen mit Infrarot-Licht und Mittel zum Aufzeichnen des erhaltenen Absorptionsspektrums; c. Mittel zum Durchführen einer Fourier-Transformation des erhaltenen Absorptionsspektrums in ein FT-IR-Spektrum; d. Mittel zum Berechnen der ersten und gegebenenfalls weiterer Ableitungen des FT-IR-Spektrums; und e. Mittel zum Vergleichen der Ableitung(en) aus Schritt (c) in einem vorgewählten Wellenzahlbereich mit derselben bzw. denselben Ableitung(en) zuvor erstellter Referenz-FT-IR-Spektren, die in der Vorrichtung gespeichert sind.

## Beschreibung

Die vorliegende Erfindung betrifft ein Verfahren zur *in-vitro* Bestimmung von ausdifferenzierten Säugerzellen, insbesondere zu deren qualitativer Bestimmung, der Typisierung, der Quantifizierung, der Zustandsanalyse und/oder Zustandskontrolle der Säugerzellen. Die Erfindung betrifft weiterhin eine Vorrichtung zur Durchführung dieses Verfahrens.

### Hintergrund der Erfindung

Verfahren zur Bestimmung, insbesondere Typisierung und Zustandskontrolle von ausdifferenzierten Säugerzellen werden insbesondere im Bereich der Transplantationsmedizin benötigt. So ist es beispielsweise möglich, Gewebsersatz dadurch herzustellen, dass man dem Empfänger eigene, intakte Zellen des entsprechenden Gewebetyps entnimmt, diese *in vitro* kultiviert und nach Erreichen der erforderlichen Zellzahl in den Patienten zurückführt. Dieses kann entweder in Form von Lösungen, gezogenen Gewebeteilen oder dadurch erfolgen, dass die Zellen auf einer vorzugsweise bioresorbierbaren Matrix kultiviert und zusammen mit der Matrix rückimplantiert werden. Entsprechende Verfahren, Matrizes und Kulturmedien sind beschrieben beispielsweise in den deutschen Anmeldungen 101 62 205.8, 101 62 960.5, 102 20 358.7, 102 22 896.5 und den dort genannten Literaturstellen.

Die Kultivierung *in vitro* erfordert zum Einen, dass aus den vom Patienten gewonnenen Gewebebiopsaten ausschließlich die gewünschten Zelltypen kultiviert wird, und zum Anderen, dass auch nur dieser Zelltyp wieder in den Patienten transplantiert wird. Hierfür ist eine strenge Kontrolle der Präparation aus dem Ausgangsmaterial, der Zellkultur wie auch des fertigen, zu transplantierenden Zellproduktes erforderlich. Ein wesentliches Problem sowohl bei der Gewinnung der Biopsate wie auch bei der Führung der Kultur besteht jedoch darin, dass die Zellen innerhalb eines Gewebes nicht in Reinform sondern in Form von Mischungen verschiedener Zelltypen vorliegen. Beispielsweise finden sich im Knorpel neben den Chondrozyten oder auch Knorpelzellen Fibroblasten, die auch in den meisten anderen Geweben anzutreffen sind. Fibroblasten stellen eine insofern unerwünschte Verunreinigung von Zellkulturen dar, als sie üblicherweise schneller als die gewünschten spezifischen ausdifferenzierten Zellen wachsen, somit schon nach kurzer Zeit die Mehrheit der Zellen in der Kultur darstellen können und diese dann nicht mehr dem gewünschten Gewebetyp entspricht.

Zur Überwachung der Transplantate war es bislang erforderlich; die Zellen dieser Kultur, wie auch des Ausgangsmaterials oder des fertigen Produkts anhand morphologischer, histologischer und genetischer Verfahren (PCR) zu bestimmen, insbesondere zu typisieren und auch zu quantifizieren. Derartige Bestimmungen erfordern jedoch neben substantieller Erfahrung und Kenntnis des Untersuchenden einen erheblichen Zeitaufwand, wie auch apparativen Aufwand für die Durchführung der verschiedenen Test. Gewünscht wäre es, für die Typisierung, ggf. auch Quantifizierung und Zustandskontrolle der zu transplantierenden Zellen ein einfaches, möglichst automatisch durchzuführendes Verfahren zu haben, mit dem die Bestimmung schnell, routinemäßig und zuverlässig erfolgen kann.

### Zusammenfassung der Erfindung

Die obige Aufgabe wird gelöst wie auch weitere Nachteile des Standes der Technik überwunden durch ein Verfahren zur *in-vitro* Bestimmung, insbesondere Typisierung ausdifferenzierter Säugerzellen, umfassend die Schritte:
a. Bestrahlen der Zellen mit Infrarot-Licht und Aufzeichnen des erhaltenen Absorptionsspektrums;
b. Durchführen einer Fourier-Transformation des Absorptionsspektrums aus (a) zum Erhalt eines FT-IR-Spektrums;
c. Berechnen der ersten und gegebenenfalls höherer Ableitungen des FT-IR-Spektrums; und
d. Vergleichen der Ableitung(en) aus Schritt (c) in einem vorgewählten Wellenzahlbereich mit derselben bzw. denselben Ableitung(en) zuvor ermittelter Referenz-FT-IR-Spektren.

Neben diesem Verfahren ist weiterer Gegenstand der Erfindung eine Vorrichtung zur *in-vitro* Bestimmung, insbesondere Typisierung ausdifferenzierter Säugerzellen, umfassend:
a. eine Probenaufnahmevorrichtung für eine Suspension der ausdifferenzierten Säugerzellen;
b. Mittel zum Bestrahlen der Zellen mit Infrarot-Licht und Mittel zum Aufzeichnen des erhaltenen Absorptionsspektrums;
c. Mittel zum Durchführen einer Fourier-Transformation des erhaltenen Absorptionsspektrums in ein FT-IR-Spektrum;
d. Mittel zum Berechnen der ersten und gegebenenfalls höherer Ableitungen des FT-IR-Spektrums; und
e. Mittel zum Vergleichen der Ableitung(en) aus Schritt (c) in einem vorgewählten Wellenzahlbereich mit derselben bzw. denselben Ableitung(en) zuvor erstellter Referenz-FT-IR-Spektren, die in der Vorrichtung gespeichert sind.

### Beschreibung der Zeichnungen

Fig. 1 stellt ein FT-IR-Spektrum von Periostzellen und Chondrozyten im Wellenzahlbereich zwischen 700 und 4.000 cm⁻¹ dar.
Fig. 2 zeigt die zweite Ableitung der Spektren aus Fig. 1 im Wellenzahlbereich von 900 bis 1.200 cm⁻¹.
Fig. 3 zeigt die Zuordnung der charakteristischen Unterschiede der Spektren aus Fig. 2 zu einzelnen Zellgruppen durch Vergleich mit einem Standard.
Fig. 4 zeigt ein Dendrogramm der verschiedenen Zelltypen, wobei die Spektren unterschiedlicher Proben anhand ihrer Heterogenität unterschiedlichen Stammbäumen zugeordnet wurden. Hieraus ist ersichtlich, dass sich anhand der Heterogenität der erhaltenen zweiten Ableitungen gemäß Fig. 2 eine eindeutige Zuordnung zu einem bestimmten Zelltyp erzielen lässt.
Fig. 5 zeigt die Zuordnungsmöglichkeit von Blindproben zu verschiedenen Zelltypen gemäß dem Verfahren nach der vorliegenden Erfindung. D:Chondrozyten; E:Melanozyten; B und F:Periostzellen.
Fig. 6 zeigt schließlich die Möglichkeit Chondrozyten auch hinsichtlich ihres Zustandes zu klassifizieren. Deutlich sichtbar ist die Unterscheidbarkeit von Chondrozyten in Puffer, enthaltend 10 % human Serumalbumin, 5% human Serumalbumin bzw. kein Serumalbumin und bei sofortiger Untersuchung *ex vivo.* Deutlich ist bei einem Vergleich der Figuren 5 und 6 auch die sehr viel geringere Heterogenität der Chondrozytenpräparationen untereinander im Vergleich zur Heterogenität zwischen verschiedenen Zelltypen sehen.

### Genaue Beschreibung der Erfindung

Die vorliegende Erfindung betrifft ein Verfahren zur *in-vitro* Bestimmung ausdifferenzierter Säugerzellen, das einfach und vorzugsweise automatisiert durchzuführen ist, schnell Ergebnisse liefert und zuverlässig die Bestimmung ausdifferenzierter Säugerzellen gestattet. So erfordern die klassischen Verfahren zur Typisierung von Zellen wie morphologische und histologische Verfahren sowie die genetische Bestimmung beispielsweise mittels PCR typischerweise einen Zeitaufwand von bis zu 48 Stunden oder länger bis konkret Ergebnisse erhalten werden können. Andererseits müssen aber dann, wenn Gewebsproben aus einem Patienten entnommen werden, die daraus gewonnenen Zellen unmittelbar in die Kultur überführt werden, um die Lebensfähigkeit der Zellen zu erhalten. Ebenso ist es erforderlich, das Transplantat, sofern denn die gewünschte Zellzahl erzielt ist, baldmöglichst in den Patienten zurückzuführen. Außerdem muss die Typisierung des zu transplantierenden Zellmaterials möglichst zeitnah vor der Transplantation erfolgen, nicht etwa mit zwei Tagen Abstand, wie dies nach dem Stand der Technik bislang erforderlich war.

Gemäß dem erfindungsgemäßen Verfahren kann die Bestimmung der ausdifferenzierten Säugerzellen, und insbesondere deren Typisierung nun einfach und zuverlässig dadurch erfolgen, dass man die Zellen oder ein Aliquot derselben mit Infrarot-Licht bestrahlt und das erhaltene Absorptionsspektrum aufzeichnet, an diesem Absorptionsspektrum eine Fourier-Transformation zum Erhalt eines FT-IR-Spektrums durchführt, die erste und/oder höhere Ableitungen des FT-IR-Spektrums errechnet und diese Ableitung oder Ableitungen in einem vorgewählten Wellenzahlbereich mit einer oder mehreren Ableitung(en) der jeweils selben Ordnung von zuvor ermittelten Referenz FT-IR-Spektren vergleicht. Die Referenz FT-IR-Spektren werden üblicherweise ausgehend von Präparationen gereinigter, ausdifferenzierter Säugerzellen mindestens eines Zelltyps erhalten, bei dem es sich vorzugsweise um den gewünschten Zelltyp handelt. So werden beispielsweise bei Knorpeltransplantaten Referenz FT-IR-Spektren an gereinigten Präparationen von Chondrozyten erhalten. Die Ableitungen der FT-IR-Spektren des zu bestimmenden Transplantats werden dann mit den Ableitungen dieses Referenz-FT-IR-Spektrums verglichen.

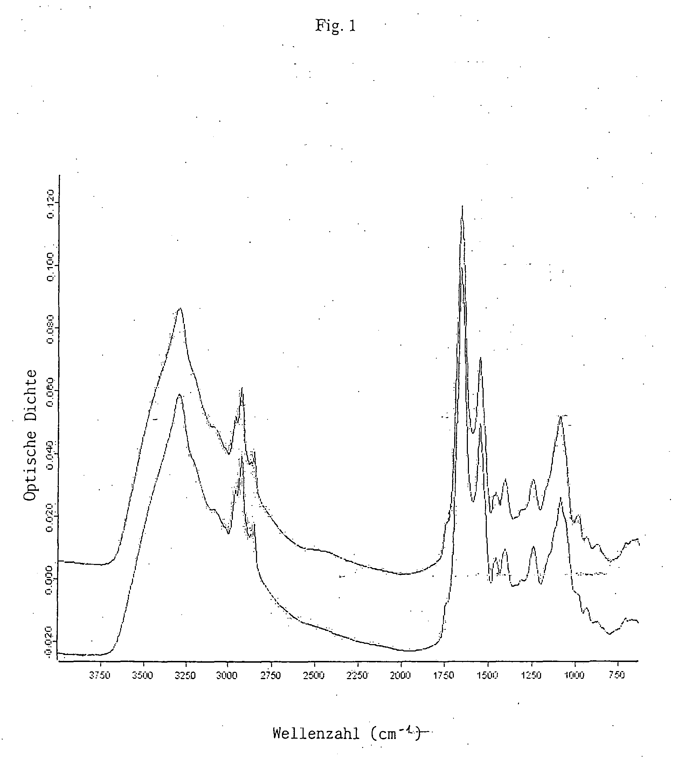

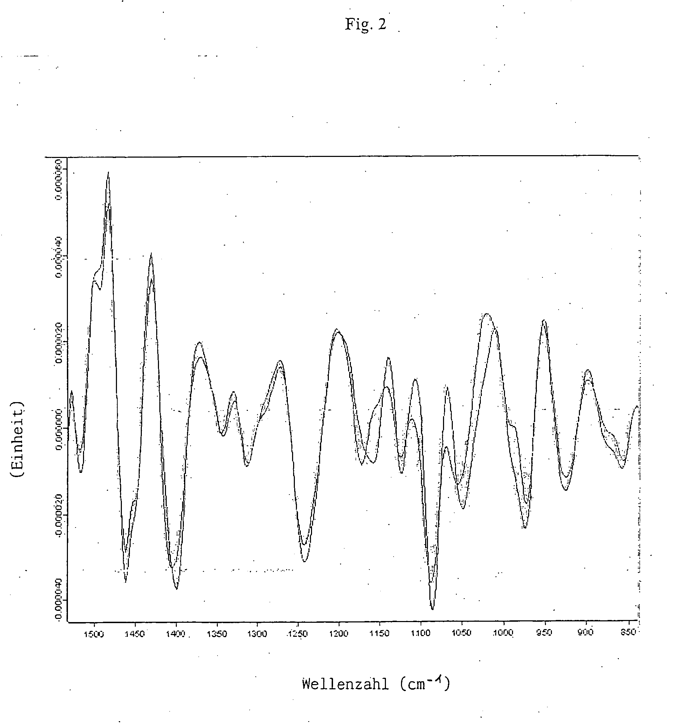

Erfindungsgemäß wird zum Bestrahlen der Zellen Infrarot-Licht geeigneter Wellenlänge verwendet. Grundsätzlich ist jedes Infrarot-Licht geeignet, d. h. kann Licht im Bereich einer Wellenlänge von 760 nm bis 500 µm verwendet werden. Infrarot-Licht des nahen, mittleren oder fernen IR ist geeignet. Vorzugsweise verwendet man Infrarot-Licht einer Wellenlänge im Bereich von 760 nm bis 2.000 nm und am meisten bevorzugt Infrarot-Licht des mittleren Bereichs von 800 nm bis 1.500 nm. Die Verwendung des mittleren IR ist deswegen bevorzugt, weil hier eine ausgeprägte Adsorption durch Kohlenhydrate zu beobachten ist. Ohne hierdurch gebunden sein zu wollen, wird vermutet, dass sich in diesem Bereich die unterschiedlichen Kohlenhydratstrukturen zeigen, die bekanntermaßen von jedem Zelltyp auf der Zelloberfläche exprimiert werden. Der Vergleich der Spektren der Kohlenhydrate gestattet somit einen Rückschluss auf den jeweils vorhandenen Zelltyp. Entsprechende IR-Spektren von Periostzellen und Chondrozyten sind für Wellenzahlen im Bereich von 700 cm⁻¹ bis 4.000 cm⁻¹ in Fig. 1 gezeigt. Aus Fig. 1 ist ersichtlich, dass sich die FT-IR-Spektren der Zellen selbst nicht wesentlich voneinander unterscheiden, die 2. Ableitungen jedoch, wie aus Fig. 2 ersichtlich, charakteristische Unterschiede aufweisen.

Erfindungsgemäß wird dementsprechend die erste Ableitung und/oder auch eine oder mehrere höhere Ableitungen dieses Spektrums zur eindeutigen Differenzierung verwendet. Die zweite Ableitung des FT-IR-Spektrums aus Fig. 1 ist in Fig. 2 für den Bereich des mittleren IR-Lichts gezeigt: Fig. 2 macht deutlich, dass sich trotz erheblicher Ähnlichkeit der eigentlichen Spektren im Bereich der zweiten wie auch von höheren Ableitungen deutliche und charakteristische Unterschiede der Spektren zeigen. Diese werden erfindungsgemäß zur Bestimmung der ausdifferenzierten Säugerzellen verwendet.

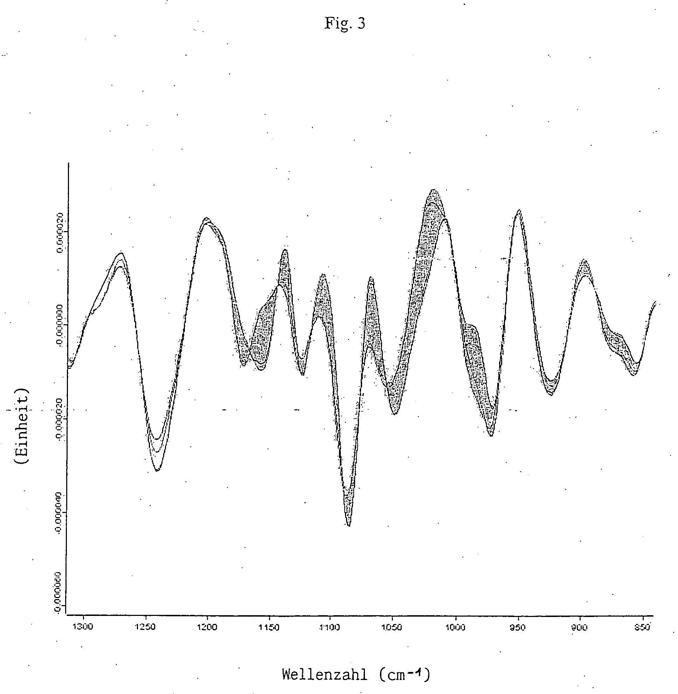

Fig. 3 zeigt diese zweiten Ableitungen im Vergleich zu einer Ableitung einer Standardchondrozytenpräparation, wobei die dunkel schraffierten Flächen Unterschiede zwischen Chondrozyten und Periostzellen darstellen, wogegen die hell schraffierten Flächen Differenzierungen unter Chondrozyten darstellen und die weißen Differenzflächen für Periostzellen stehen. Die Heterogenitäten der Spektren lassen sich durch Clusteranalyse oder Differenzbildung berechnen und in Form eines sogenannten Dendrogrammes darstellen. Diese Dendrogramme können durch bekannte Algorithmen ermittelt werden. Beispielsweise sind solche Algorithmen bekannt aus dem Manual der Software "Opus Version 4.2, Spectroscopic Software der Fa. Bruker GmbH, dessen diesbezügliche Offenbarung in diese Anmeldung mit aufgenommen wird. Je ähnlicher sich die Zellen sind, desto geringer ist ihre Heterogenität. Die Heterogenität zweier Zellen lässt sich im Dendrogramm aus dem kürzesten Verbindungsweg ablesen.

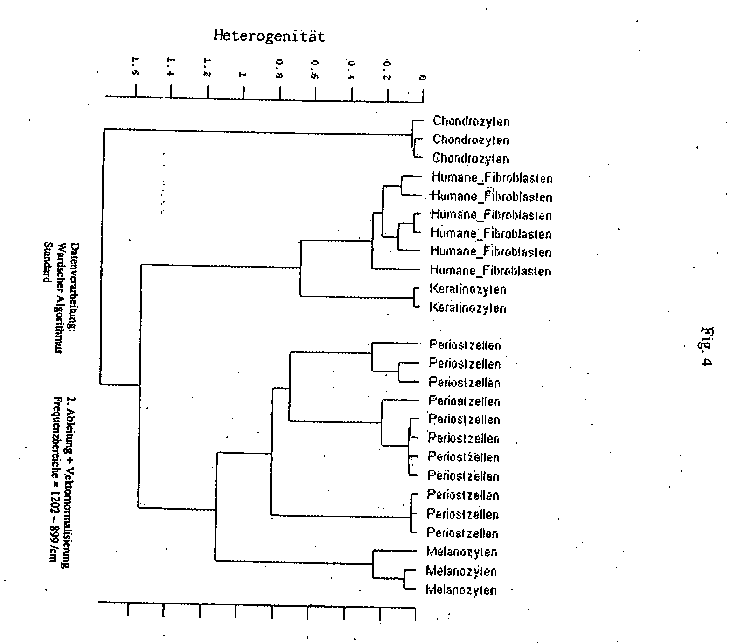

In Fig. 4 ist ein entsprechendes Dendrogramm für Chondrozyten, humane Fibroblasten, Cheratinozyten, Periostzellen und Melanozyten gezeigt. Daraus ergibt sich, dass die Heterogenität zwischen Chondrozyten und allen anderen Zelltypen etwa 1.7 beträgt, wogegen die Heterogenität zwischen humanen Fibroblasten und Kerationozyten lediglich bei etwa 0.7 liegt. Je höher die Heterogenität desto sicherer lassen sich die Zelltypen voneinander unterscheiden.

Zur Verbesserung der Auflösung können vorzugsweise mehrere Proben desselben Zelltyps, die gleich aufgearbeitet wurden, vermessen und die erhaltenen Spektren normiert werden. Normierung, Fourier-Transformation und Ableitung erfolgen nach dem Fachmann bekannten Methoden. Vorzugsweise werden alle Berechnungen computerisiert durchgeführt. Erfindungsgemäß können Ableitungen der zweiten und ggf. höherer Ordnung verwendet werden. Bevorzugt werden Ableitungen der zweiten Ordnung (zweite Ableitungen) verwendet.

Das Verfahren der vorliegenden Erfindung kann vor Schritt (a) zusätzlich umfassen:
(i) Auftragen einer Suspension der zu bestimmenden Zellen auf einen geeigneten Träger; und
(ii) Verdampfen des Lösungsmittels aus-der Suspension zum Erhalt einer trockenen Probe.

Das erfindungsgemäße Verfahren wird üblicherweise an Zellen in Suspension durchgeführt. Hierzu können die Zellen entweder direkt aus dem Gewebeverband herausgelöst und der erfindungsgemäßen Bestimmung unterworfen werden. Alternativ können die Zellen zunächst kultiviert und dann Aliquots der Kultur, ggf. nach Trypsinierung zur Ablösung der kultivierten Zellen von ihrem Untergrund, mit Hilfe des erfindungsgemäßen Verfahrens untersucht werden. Wahlweise können Aliquots der Probenlösung auch zunächst eingefroren und zur Untersuchung wieder aufgetaut werden. Üblicherweise reichen bei Zellzahlen von 1 x 10³ bis 2 x 10⁵ Zellen pro Milliliter 10 bis 500 µl der Zellsuspension für die Bestimmung aus. Die Zellen können in einem geeigneten Lösungsmittel gelöst sein, beispielsweise Wasser, Kulturmedium, Puffer, physiologische Kochsalzlösung usw. Es ist nicht erforderlich, aus diesem Lösungsmittel Zusatzstoffe wie beispielsweise Serumalbumine, insbesondere Humanserum usw. abzutrennen.

Die Suspension wird auf einen für die IR-Messung geeigneten Träger aufgetragen. Vorzugsweise werden mindestens drei, üblicherweise drei bis fünf parallele Messungen an einer Probe durchgeführt. Nach dem Auftrag lässt man das Lösungsmittel verdampfen, so dass eine trockene Probe erhalten wird. Diese wird erfindungsgemäß mit IR-Licht bestrahlt und das Adsorptionsspektrum gemäß dem erfindungsgemäßen Verfahren aufgezeichnet. Der Träger kann aus jedem IR-geeigneten Material bestehen. Vorzugsweise handelt es sich um Glas oder Silizium. Geeignete Träger sind beispielsweise in der deutschen Patentanmeldung DE 100 60 560 beschrieben.

In Schritt (d) des erfindungsgemäßen Verfahrens werden die von der Probe erhaltenen - Ableitungen der FT-IR-Spektren mit derselben Ableitung eines zuvor ermittelten Referenz-FT-IR-Spektrum verglichen. Für diesen Vergleich werden die Referenzspektren entweder ausgehend von Reinkulturen des interessierenden Zelltyps und/oder ausgehend von bekannten Mischkulturen des interessierenden Zelltyps mit einem oder mehreren weiteren Zelltypen erhalten. Vorzugsweise werden die Referenzspektren ausgehend von Reinkulturen des interessierenden Zelltyps in variierenden Lösungsmitteln erstellt. Eines dieser Lösungsmittel ist mit dem Lösungsmittel der Probe identisch.

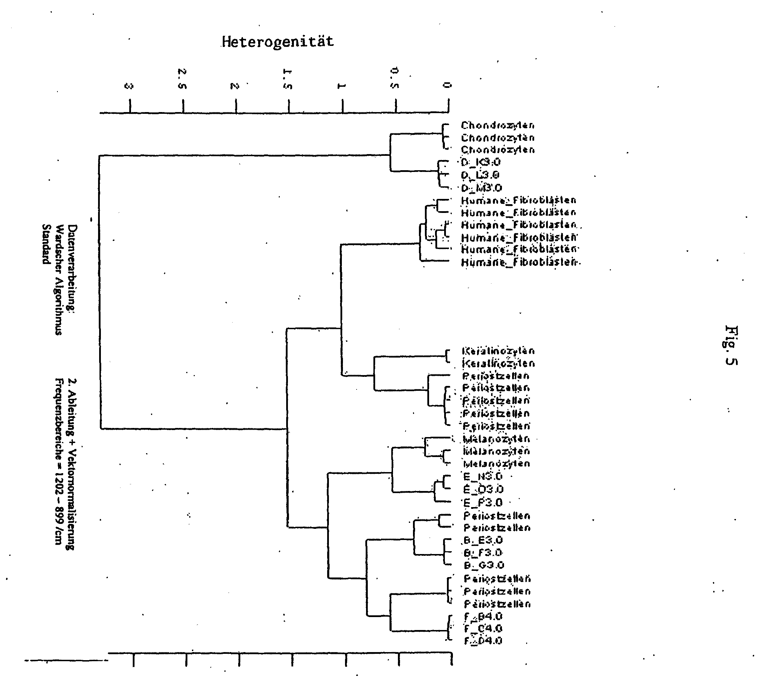

Die Variation des Lösungsmittels gestattet es, wie aus Fig. 5 ersichtlich, auch die Lösungsmittelumgebung des fraglichen Zelltypes zu bestimmen. Beispielsweise können mit Hilfe des erfindungsgemäßen Verfahrens Suspensionen von Chondrozyten mit 10% Zusatz an humanem Serumalbumin von Suspensionen desselben Zelltyps mit einem Zusatz von nur 5% humanem Serumalbumin oder solchen ohne Serumalbumin unterschieden werden. Die Erfindung gestattet somit nicht nur die Bestimmung des Zelltyps sondern auch eine Zustandskontrolle der Säugerzellen hinsichtlich ihres Umgebungsmilieus.

Wahlweise können die Referenzspektren auch ausgehend von Mischkulturen des interessierenden Zelltyps mit einem oder mehreren weiteren Zelltypen erstellt werden. Beispiele hierfür sind Mischkulturen der gewünschten Zellen mit beispielsweise den ubiquitär kontaminierenden Fibroblasten. Im Vergleich zu solchen Misch-Referenz-Spektren lässt sich somit über das erfindungsgemäße Verfahren die Kontamination einer Zellkultur bzw. Probe und ggf. das Verhältnis von gewünschtem Zelltyp zu kontaminierendem Zelltyp bestimmen.

Mit Hilfe des erfindungsgemäßen Verfährens lassen sich ausdifferenzierte Säugerzellen bestimmen. Hierbei handelt es sich vorzugsweise um humane Gewebezellen, insbesondere Binde- und Stützgewebezellen. Am meisten bevorzugt werden die Zellen ausgewählt aus der Gruppe, bestehend aus Chondrozyten, Osteozyten, Periostzellen, und Epithelzellen. Im Rahmen von Mischkulturen werden üblicherweise Fibroblasten als weiterer Zelltyp zugesetzt. Das erfindungsgemäße Verfahren kann zur qualitativen Bestimmung, Typisierung, Quantifizierung und/oder Zustandskontrolle genutzt werden.

Die Durchführung des erfindungsgemäßen Verfahrens erfolgt üblicherweise an einer Vorrichtung zur *in-vitro* Bestimmung, insbesondere Typisierung ausdifferenzierter Säugerzellen, die umfasst:
a. eine Probenaufnahmevorrichtung für eine Suspension der ausdifferenzierten Säugerzellen;
b. Mittel zum Bestrahlen der Zellen mit Infrarot-Licht und Mittel zum Aufzeichnen des erhaltenen Absorptionsspektrums;
c. Mittel zum Durchführen einer Fourier-Transformation des erhaltenen Absorptionsspektrums in ein FT-IR-Spektrum;
d. Mittel zum Berechnen der ersten und gegebenenfalls höherer Ableitungen des FT-IR-Spektrums; und
e. Mittel zum Vergleichen der Ableitung(en) aus Schritt (d) in einem vorgewählten Wellenzahlbereich mit derselben bzw. denselben Ableitung(en) zuvor erstellter Referenz-FT-IR-Spektren, die in der Vorrichtung gespeichert sind.

Vorrichtungen gemäß der Merkmale a bis d sind im Stand der Technik bekannt. Hierzu wird beispielsweise auf die deutschen Patentschriften DE 199 49 953 oder DE 199 40 981 verwiesen, deren Offenbarung durch Bezugnahme hierin aufgenommen wird.

Die erfindungsgemäßen Vorrichtungen sind dadurch gekennzeichnet, dass die Referenzspektren bereits in der Vorrichtung gespeichert sind und somit ein automatisierter Vergleich der gewünschten Ableitungen mit den Ableitungen der Referenz FT-IR-Spektren automatisiert möglich ist.

### Beispiele

### Beispiel 1

Zur Durchführung des erfindungsgemäßen Verfahrens wurde ein Infrarot-Messgerät der Marke HTS-XT der Firma Bruker Optik GmbH, Deutschland, verwendet. Hierbei handelt es sich um ein IR-Messgerät für ein standardisiertes Mikrotiterplattenformat, welches bereits Software für die Messung, Auswertung, d. h. Fourier-Transformation, und Dokumentation sowie Ableitung der aufgezeichneten Absorptionsspektren enthält. Für die Messungen wurden Mikrotiterplatten aus Silizium verwendet, welche sowohl die Transmission als auch Reflektion des Lichtes erlauben. Die Messungen erfolgten mit folgenden Paramtern: Tensor 27; Auflösung 4 cm⁻¹; Messzeit: 53 sec je Probe (64 Scans).

Auf die einzelnen Messpunkte der Mikrotiterplatte wurden drei bis vier Mikroliter der Zellsuspensionen aufgetragen, wober drei parallele Bestimmungen für jede Probe durchgeführt wurden.

Bei den Proben handelt es sich um eingefrorene und wiederum aufgetaute Zellpellets von Chondrozyten in PBS mit und ohne 5 oder 10 Gew.-% human Serumalbumin. Diese Zellpellets wurden mit einer Zellanzahl zwischen 5 x 10⁵ bis 5 x 10⁶ Zellen eingefroren, bei -20°C gelagert, aufgetaut, vorsichtig geschüttelt und dann direkt aufgetragen. Nach dem Auftrag ließ man diese Suspensionen ca. 30 Minuten bei Raumtemperatur trocknen. Die Messung erfolgte unmittelbar nach dem Trocknen in einem Wellenlängenbereich von 700 bis 4.000 nm (Wellenzahl 750 cm⁻¹ bis 3.750 cm⁻¹).

Die gewonnenen Spektren von Periostzellen und Chondrozyten (ohne Serum) sind in Fig. 1 und die 2. Ableitung in Fig. 2 gezeigt. Ähnliche Messungen wurden mit humanen Fibroblasten, Keratinozyten und Melanozyten zur Typisierung der verschiedenen Zelltypen in einem Wellenzahlbereich von 900 cm⁻¹ bis 1.200 cm⁻¹ durchgeführt. Das erhaltene Dendrogramm ist in Fig. 4 gezeigt.

### Beispiel 2

Die in Beispiel 1 gewonnenen Dendrogramme und Spektren als Referenzspektren verwendend wurden anschließend identisch aufbereitete Blindproben untersucht. Diese ließen sich, wie aus Fig. 5 ersichtlich eindeutig ihrem jeweiligen Zelltyp nämlich Chondrozyten, Melanozyten und Periostzellen zuordnen.

### Beispiel 3

Gemäß Beispiel 1 wurden Chondrozytenproben mit 10 % humanem Serumalbumin, 5% humanem Serumalbumin, ohne Serumalbumin wie auch *ex vivo* Proben, d. h. Chondrozytensuspensionen unmittelbar nach Herstellung derselben, ohne zwischenzeitiges Einfrieren, untersucht.

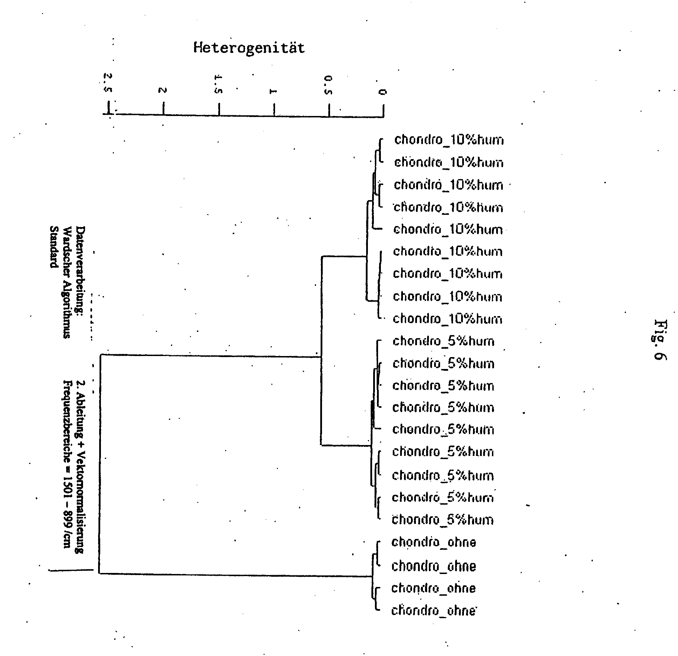

Das entsprechende Dendrogramm ist in Fig. 6 gezeigt. Aus dem Dendrogramm ist ersichtlich, dass sich über das erfindungsgemäße Verfahren auch der Zustand der Chondrozyten bzw. ihre unmittelbare Lösungsumgebung bestimmen lässt.

Die vorliegende Erfindung stellt somit ein einfaches, schnelles Verfahren zur Bestimmung, insbesondere Typisierung von ausdifferenzierten Säugerzellen bereit, das beispielsweise als Routinekontrolle für in der Transplantationsmedizin zu verwendende Zellzubereitungen geeignet ist. Dieses ist insbesondere deshalb möglich, weil mit minimalen Probenmengen eine schnelle Typisierung ohne großen Aufwand möglich ist.

Die vorliegende Anmeldung ist nicht auf den Gegenstand der gegebenen Beispiele beschränkt. Der Fachmann wird erkennen, dass Modifikationen des Verfahrens möglich sind, wobei der Schutzbereich der Anmeldung lediglich durch die angefügten Ansprüche beschränkt sein soll.

## Patentansprüche

1. Verfahren zur *in-vitro* Bestimmung, insbesondere Typisierung ausdifferenzierter Säugerzellen, umfassend die Schritte:
a. Bestrahlen der Zellen mit Infrarot-Licht und Aufzeichnen des erhaltenen Absorptionsspektrums;
b. Durchführen einer Fourier-Transformation des Absorptionsspektrums aus (a) zum Erhalt eines FT-IR-Spektrums;
c. Berechnen der ersten und gegebenenfalls höherer Ableitungen des FT-IR-Spektrums; und
d. Vergleichen der Ableitung(en) aus Schritt (c) in einem vorgewählten Wellenzahlbereich mit derselben bzw. denselben Ableitung(en) zuvor ermittelter Referenz-FT-IR-Spektren.

2. Verfahren nach Anspruch 1, worin die Referenz-FT-IR-Spektren ausgehend von Präparationen gereinigter ausdifferenzierter Säugerzellen mindestens eines Zelltyps erhalten werden.

3. Verfahren nach Anspruch 1 oder 2, bei dem man Infrarot-Licht mit einer Wellenlänge im Bereich von 760 nm bis 500 µm, vorzugsweise 760 nm - 2000 nm, und am meisten bevorzugt 800 nm bis 1500 nm verwendet.

4. Verfahren nach einem der vorstehenden Ansprüche, vor Schritt (a) zusätzlich umfassend die Schritte:
(i) Auftragen einer Suspension der zu bestimmenden Zellen auf einen geeigneten Träger; und
(ii) Verdampfen des Lösungsmittels aus der Suspension zum Erhalt einer trockenen Probe.

5. Verfahren nach einem der vorstehenden Ansprüche, bei dem für den Vergleich in Schritt (c) Referenzspektren ausgehend von Reinkulturen des interessierenden Zelltyps und/oder von bekannten Mischkulturen des interessierenden Zelltyps mit einem oder mehreren weiteren Zelltypen erstellt werden.

6. Verfahren nach dem vorstehenden Anspruch, worin die Referenzspektren ausgehend von Reinkulturen des interessierenden Zelltyps in variierenden Lösungsmitteln erstellt werden.

7. Verfahren nach einem der Ansprüche 1 bis 5, bei dem die Referenzspektren ausgehend von Mischkulturen des interessierenden Zelltyps erstellt werden.

8. Verfahren nach einem der vorstehenden Ansprüche, bei dem die Bestimmung auf Basis der zweiten Ableitung des FT-IR-Spektrums erfolgt.

9. Verfahren nach einem der vorstehenden Ansprüche, worin es sich bei den Säugerzellen um Zellen handelt, ausgewählt aus der Gruppe bestehend aus Chondrozyten, Osteozyten, Periostzellen, Epithelzellen, insbesondere Hautzellen wie Melanozyten, Fibroblasten und Muskelzellen.

10. Verfahren nach Anspruch 5 und 7, worin die Mischkultur als weiteren Zelltyp Fibroblasten enthält.

11. Verfahren nach einem der vorstehenden Ansprüche, bei dem die Schritte (a) bis (d) automatisch durchgeführt werden.

12. Verfahren nach einem der vorstehenden Ansprüche, durchgeführt zu qualitativen Bestimmung, Typisierung, Quantifizierung und/oder Zustandskontrolle der Säugerzellen.

13. Verfahren nach einem der vorstehenden Ansprüche worin aus dem Vergleich der Ableitungen ein Heterogenitätswert zu verschiedenen Referenz-FT-IR-Spektren unterschiedlicher Zelltypen errechnet und die Typisierung durch Einordnung in ein Dendrogramm erfolgt.

14. Vorrichtung zur *in-vitro* Bestimmung, insbesondere Typisierung ausdifferenzierter Säugerzellen, umfassend:
a. eine Probenaufnahmevorrichtung für eine Suspension der ausdifferenzierten Säugerzellen;
b. Mittel zum Bestrahlen der Zellen mit Infrarot-Licht und Mittel zum Aufzeichnen des erhaltenen Absorptionsspektrums;
c. Mittel zum Durchführen einer Fourier-Transformation des erhaltenen Absorptionsspektrums in ein FT-IR-Spektrum;
d. Mittel zum Berechnen der ersten und gegebenenfalls weiterer Ableitungen des FT-IR-Spektrums; und
e. Mittel zum Vergleichen der Ableitung(en) aus Schritt (c) in einem vorgewählten Wellenzahlbereich mit derselben bzw. denselben Ableitung(en) zuvor erstellter Referenz-FT-IR-Spektren, die in der Vorrichtung gespeichert sind.

15. Vorrichtung nach Anspruch 14, bei dem Infrarot-Licht mit einer Wellenlänge im Bereich von 760 nm bis 500 µm, vorzugsweise 760 nm - 2000 nm, und am meisten bevorzugt 800 nm bis 1500 nm verwendet wird.
